# EUROPÄISCHE PATENTSCHRIFT

(11) **EP 1 423 086 B1**
(45) Veröffentlichungstag und Bekanntmachung des Hinweises auf die Patenterteilung: **08.08.2007**
(21) Anmeldenummer: 02767437.3
(22) Anmeldetag: 27.08.2002
(51) Int. Cl.: A61Q 17/02, A61K 8/37

(54) **STABILISIERUNG KOSMETISCHER ODER DERMATOLOGISCHER ZUBEREITUNGEN, WELCHE REPELLENT-WIRKSTOFFE ENTHALTEN**
STABILISATION OF COSMETIC OR DERMATOLOGICAL PREPARATIONS CONTAINING REPELLENT ACTIVE INGREDIENTS
STABILISATION DE PREPARATIONS COSMETIQUES OU DERMATOLOGIQUES CONTENANT DES SUBSTANCES ACTIVES REPULSIVES

(30) Priorität: 29.08.2001 DE 10141471
(43) Veröffentlichungstag der Anmeldung: 02.06.2004
(73) Patentinhaber: Beiersdorf AG, 20245 Hamburg (DE)
(72) Erfinder: WENDEL, Volker, 20255 Hamburg (DE); GÖPPEL, Anja, 22527 Hamburg (DE); SUCKERT, Anja, 20259 Hamburg (DE)
(86) Internationale Anmeldenummer: PCT/EP2002/009543
(87) Internationale Veröffentlichungsnummer: WO 2003/020232

(56) Entgegenhaltungen:
- EP-A- 0 962 135
- EP-A- 1 092 413
- EP-A- 1 129 696
- WO-A-01/89452
- WO-A-01/89465
- FR-A- 2 801 208
- FR-A- 2 801 209
- GB-A- 660 131
- BONDA C ET AL: "A NEW PHOTOSTABILIZER FOR FULL SPECTRUM SUNCREENS" COSMETICS & TOILETRIES, WHEATON, IL, US, Bd. 115, Nr. 6, 2000, Seiten 37-45, XP001010090 ISSN: 0361-4387
- DATABASE WPI Derwent Publications Ltd., London, GB; AN 1993-162085 XP002116426 & JP 05 092905 A (SHISEIDO) 16. April 1993 (1993-04-16)

## Beschreibung

Die vorliegende Erfindung betrifft die Verwendung von Alkylnaphthalaten zur Erhöhung der Stabilität kosmetischer oder dermatologischer Zubereitungen, welche Insektenrepellentien enthalten. Bevorzugt betrifft die vorliegende Erfindung kosmetische oder dermatologische Zubereitungen zur gezielten Verhinderung von Insektenstichen, in welchen derartige Wirkstoffe über einen langen Zeitraum stabil gelagert werden können und welche ein gutes Transportmittel für diese Wirkstoffe darstellen.

Insektenabwehrmittel (Insektenvertreibungsmittel, Insektenschutzmittel, Repellentien, Repellents) sind Präparate, die zur Abwehr und/oder Vertreibung von Insekten, aber auch Zecken und Milben äußerlich angewendet werden und verhindern sollen, daß diese auf der Haut aktiv werden. Insektenabwehrmittel sollen die Haut vor Belästigung durch blutsaugende oder beißende Insekten und andere Parasiten und/oder Lästlinge schützen, indem sie diese abwehren, bevor sie auf die Haut fliegen, so daß es nicht zu Stichen oder Bissen kommt. Die Mittel wirken dementsprechend nicht als Kontaktgifte, sondern nur als Abwehrmittel, da sie die Tiere nicht töten, sondern lediglich vertreiben.

Demgemäß sind im Sinne der vorliegenden Erfindung unter dem Begriff "Insektenabwehrmittel" nicht nur solche Formulierungen zu verstehen, die gegen Insekten wirksam sind. Vielmehr gilt das nachstehend Gesagte selbstverständlich auch für solche Präparate, die andere blutsaugende oder beißende Parasiten und/oder Lästlinge abwehren oder vertreiben, auch wenn dies im Einzelfall nicht erwähnt sein mag.

Schon seit Urzeiten werden die Menschen von stechenden oder beißenden Insekten oder anderen Parasiten geplagt. Dementsprechend alt ist das Bedürfnis der Menschheit nach Insektenabwehrmitteln. Eine schon seit der Frühgeschichte bekannte Methode, lästigen oder schädlichen Insekten ihren Aufenthalt in der Nähe des Menschen unattraktiv oder unangenehm zu machen, ist das Anzünden von Feuern mit aromatisch oder streng riechenden Kräutern oder Hölzern und starker Rauchentwicklung. Auch die Behandlung der Haut mit stark riechenden Substanzen zur Abwehr von Insekten ist bereits seit der Antike bekannt. Um die letzte Jahrhundertwende war eine Reihe natürlicher etherischer Öle als Insektenabwehrmittel im Gebrauch, so beispielsweise Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl.

Wegen ihrer trotz intensiven Geruchs überwiegend unzureichenden Wirksamkeit und ihrer zum Teil mangelnden Verträglichkeit in höheren Konzentrationen wurden diese Stoffe in heutigen Insektenabwehrmitteln weitgehend durch besser wirksame synthetische Substanzen verdrängt. Es handelt sich dabei überwiegend um hochsiedende Flüssigkeiten oder niedrig schmelzende kristalline Stoffe, die bei Raumtemperatur langsam verdampfen. Die meisten Repellent-Wirkstoffe gehören den Stoffklassen der Amide, Alkohole, Ester und Ether an. Dokument EP1092913 offenbart Lichtschutz zusammensetzungen mit einem Gehalt an Insektenrepellentien. Dokument JP05092905 offenbart ester-Stabilisierte Repellent-enthaltende Zusammensetzungen.

Repellent-Wirkstoffe sollen die folgenden Bedingungen erfüllen:
Sie dürfen nicht zu schnell verdunsten und nicht in die Haut eindringen. Sie dürfen auf die Haut weder primär irritierend noch sensibilisierend wirken und sollen außerdem nicht toxisch sein. Ihre Wirksamkeit muß auch unter Einwirkung von Hautflüssigkeit und/oder UV-Strahlung erhalten bleiben.

Von den heute in Insektenabwehrmitteln ca. 15 häufig eingesetzten Wirkstoffen wird das N,N-Diethyl-3-methylbenzamid (DEET), welches sich durch die folgende Strukturformel auszeichnet 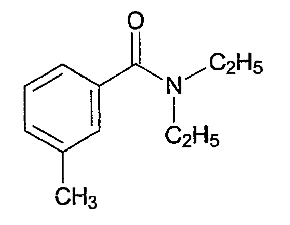 als bestes Allround-Repellent bezeichnet. Es wirkt abwehrend gegen Stechmücken, Bremsen, Sandfliegen, Zecken, Stechfliegen, Milben, Flöhe und Wanzen, wobei die Wirkungsdauer - wie bei allen Repellent-Wirkstoffen - unterschiedlich lang gegenüber den verschiedenen Spezies ist. Handelsübliche DEET-Präparate beispielsweise sind ca. 6 bis 8 Stunden gegen Mücken wirksam, jedoch nur ca. 2 bis 4 Stunden gegen Zecken.

Ein weiterer gebräuchlicher Repellent-Wirkstoff ist der 3-(N-n-Butyl-N-acetyl-amino)propionsäureethylester (auch als Repellent 3535 bezeichnet), welcher sich durch die folgende Strukturformel auszeichnet

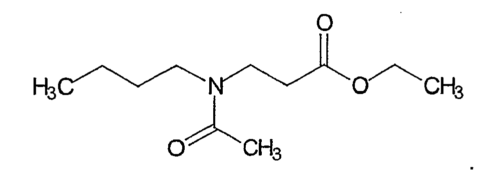

Repellent 3535 ist gegen Stechmücken (Aedes aegypti, Anopheles albimanus), Tsetsefliegen (Glossinae) und Bremsen (Tabanidae) wirksam.

Ferner gebräuchlich ist Dimethylphthalat (Palatinol M, DMP) 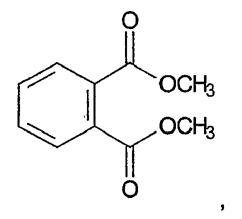 welches gegen Stechmücken (insbesondere Aedes- und Anopheles-Arten), Läuse, Zecken und Milben wirksam ist, allerdings vorwiegend in Kombination mit weiteren Repellent-Wirkstoffen eingesetzt wird.

Stiche oder Bisse von Insekten und anderen Parasiten führen normalerweise allenfalls zu Quaddelbildung, Rötung und Juckreiz sowie in vereinzelten Fällen zu meist harmlos verlaufenden Infektionen. Insekten, insbesondere Mücken können aber auch Überträger parasitärer und viraler Infektionen (wie z. B. Malaria, Gelbfieber oder Dengue-Fieber) sein. Insgesamt gibt es z. B. nicht weniger als 3000 verschiedene Stechmückenarten, von denen etwa 100 Seuchen verbreiten können. Die Abwehr oder Vertreibung dieser Insekten dient daher insbesondere auch dem Schutz vor solchen Infektionen.

Insektenabwehrmittel werden in Form von Lösungen, Gelen, Stiften, Rollern, Pump-Sprays und Aerosol-Sprays angeboten, wobei Lösungen und Sprays den Hauptteil der im Handel erhältlichen Produkte bilden. Basis für diese beiden Produktformen sind meist alkoholische bzw. wäßrig-alkoholische Lösungen unter Zusatz fettender Substanzen und leichter Parfümierungen. Andere Zubereitungsformen, wie insbesondere Emulsionen, Cremes, Salben und dergleichen, sind zwar prinzipiell denkbar, allerdings teilweise schwierig stabil zu formulieren.

Die Wirkungsdauer der Präparate nimmt üblicherweise mit der Konzentration des insektenabwehrenden Wirkstoffes im Fertigprodukt zu, welche in der Regel zwischen 20 und 70 Gew.-% beträgt. Sie ist ferner von der Schichtdicke beim Auftragen abhängig sowie von der Intensität der Schweißabsonderung und der Außentemperatur.

Ein Nachteil insbesondere von Emulsionen ist allerdings oft deren mangelnde Stabilität gegenüber höheren Repellent-Wirkstoffkonzentrationen, was sich in Phasentrennung äußert.

Es war daher Aufgabe der vorliegenden Erfindung, kosmetische oder dermatologische Zubereitungen, insbesondere Emulsionen, zu finden, welche gegenüber erhöhten Repellent-Wirkstoffkonzentrationen stabil sind.

Es war überraschend und für den Fachmann nicht vorauszusehen, daß kosmetische und dermatologische Formulierungen mit mindestens einem Repellent-Wirkstoff, dadurch gekennzeichnet, daß sie mindestens ein Dialkylnaphthalat, welches sich durch die Strukturformel 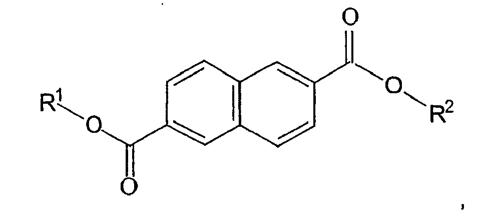 auszeichnet, worin R¹ und R² unabhängig voneinander gewählt werden aus der Gruppe der verzweigten und unverzweigten Alkylgruppen mit 6 bis 24 Kohlenstoffatomen, enthalten, den Nachteilen des Standes der Technik abhelfen.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion, eine Feststoff-Emulsionen (d. h. eine Emulsion, welche durch Feststoffe stabilisiert ist, z. B. eine Pickering-Emulsion), eine sprühbare Emulsion oder eine Hydrodispersion sein.

Die erfindungsgemäßen Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische und kosmetische Eigenschaften, wie beispielsweise die Verteilbarkeit auf der Haut oder das Einzugsvermögen in die Haut, und zeichen sich ferner durch eine sehr gute Wirkstoffeffektivität bei gleichzeitig hervorragenden Hautpflegedaten aus.

Es war für den Fachmann nicht vorauszusehen gewesen, daß die Verwendung von Alkylnaphthalaten kosmetische oder dermatologische Zubereitungen, welche mindestens einen Repellent-Wirkstoff enthalten, besser gegen den Zerfall stabilisieren würde.

Gegenstand der Erfindung ist daher auch die Verwendung von mindestens einem Dialkylnaphthalat, welches sich durch die Strukturformel 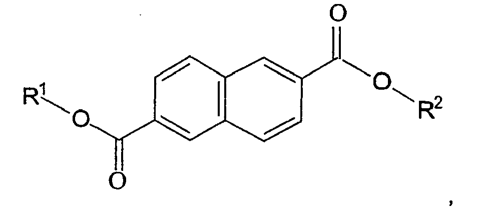 auszeichnet, worin R¹ und R² unabhängig voneinander gewählt werden aus der Gruppe der verzweigten und unverzweigten Alkylgruppen mit 6 bis 24 Kohlenstoffatomen, zur zur Erhöhung der Stabilität von kosmetischen oder dermatologischen Zubereitungen, welche mindestens einen Repellent-Wirkstoff enthalten.

Besonders vorteilhafte Repellent-Wirkstoffe im Sinne der vorliegenden Erfindung sind die obengenannten Wirkstoffe N,N-Diethyl-3-methylbenzamid, 3-(N-n-Butyl-N-acetyl-amino)-propionsäureethylester und Dimethylphthalat.

Erfindungsgemäß bevorzugt sind ferner die im folgenden aufgelisteten Repellent-Wirkstoffe:

| **Chemische Bezeichnung** | **Handels-name** | **Struktur** | **Wirksamkeit** (Literatur und Herstellerangaben) |
|---|---|---|---|
| Butopyronoxyl | Indalone | 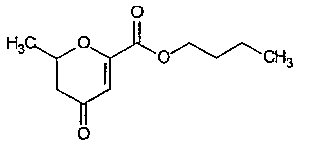 | beißende Insekten¹ |
| 2,3;4,5-bis-(2-Butylen)-tetra-hydro-2-fural-dehyd | MGK-Repellent 11 | 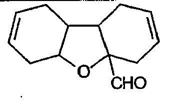 | Schaben und beißende Insekten ¹ |
| N,N-Caprylsäurediethylamid | Repellent 790 | 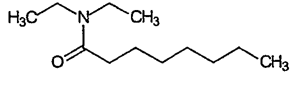 | Schaben, Stechmücken, Stubenfliege, Bremsen, Ameisen, Spinnentiere |
| o-Chlor-N,N-diethylbenzamid in Mischung mit N,N-Diethylbenzamid | Kik-Repellent | 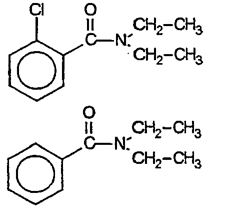 | Stechmücken, Bremsen, Flöhe, Wanzen, Zecken, Fliegen, Läuse |
| Dimethylcarbat | Dimalone | 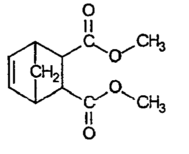 | Stechmücken, insbesondere Aedes-Arten ¹ |
| Di-n-propyliso-cinchomeronat | MGK-Repellent 326 | 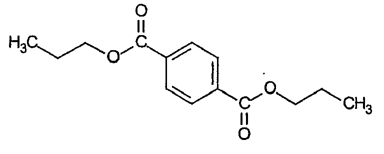 | Hausfliege, Buschfliege ¹ |
| 2-Ethylhexan-1,3-diol | Rutgers 612 | 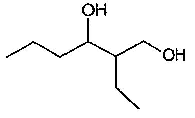 | Stechmücken, Bremsen, Fliegen, Flöhe, Milben¹ |
| N-Octyl-bicycloheptendicarboximid | MGK 264 Insecticidesynergist | 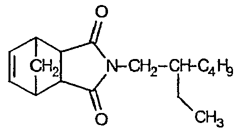 | Synergist² |
| Piperonyl-butoxid | PBO | 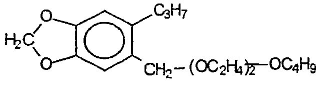 | Synergist² |

| | | | |
|---|---|---|---|
| ¹ vorwiegend in Mischung bzw. Kombination mit anderen Repellents ² wirkt als Synergist bei verschiedenen Repellents | | | |

Es ist erfindungsgemäß bevorzugt, den Gehalt der Repellent-Wirkstoff(e) (eine oder mehrere Verbindungen) in den kosmetischen oder dermatologischen Zusammensetzungen aus dem Bereich von 1 bis 70 Gew.-%, insbesondere 1 bis 50 Gew.-% zu wählen, jeweils bezogen auf das Gesamtgewicht der Zusammensetzung.

Vorteilhaft im Sinne der vorliegenden Erfindung sind Dialkylnaphthalate, für die R¹ und/oder R² verzweigte Alkylgruppen mit 6 bis 10 Kohlenstoffatomen darstellen. Ganz besonders bevorzugt im Sinne der vorliegenden Erfindung ist Diethylhexylnaphthalat, welches beispielsweise unter der Handelsbezeichnung Hallbrite TQ^{™} von CP Hall oder Corapan TQ ^{™} von H&R erhältlich ist.

Erfindungsgemäß vorteilhaft enthalten kosmetische oder dermatologische Zubereitungen 0,001 bis 20 Gew.-%, vorzugsweise 0,01 bis 15 Gew.-%, ganz besonders bevorzugt 3 bis 10 Gew.-% eines oder mehrerer Dialkylnaphthalate.

Die erfindungsgemäßen kosmetischen oder dermatologischen Formulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.%, bezogen auf das Gesamtgewicht der Formulierung, zu wählen.

Es ist insbesondere vorteilhaft, wenn die kosmetischen Zubereitungen gemäß der vorliegenden Erfindung weitere kosmetische oder dermatologische Wirkstoffe enthalten, wobei bevorzugte Wirkstoffe Antioxidantien sind, welche die Haut vor oxidativer Beanspruchung schützen können.

Vorteilhafte weitere Wirkstoffe sind natürliche Wirkstoffe und/oder deren Derivate, wie z. B. Ubichinone, Retinoide, Carotinoide, Kreatin, Taurin und/oder β-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel® 1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 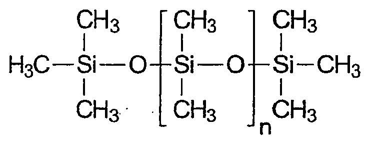 werden auch als Polydimethylsiloxan bzw. Dimethicon (INCl) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCl: Phenyl Dimethicone, Phenyl Trimethicone), cyclische Silikone (Octamethylcyclotetrasiloxan bzw. Decamethylcyclopentasiloxan), welche nach INCl auch als Cyclomethicone bezeichnet werden, aminomodifizierte Silikone (INCl: Amodimethicone) und Silikonwachse, z. B. Polysiloxan-Polyalkylen-Copolymere (INCl: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz und/oder ein oder mehrere Silikonderivate als weitere Phase enthalten. Ölfreie Formulierungen im Sinne der vorliegenden Erfindung können vorteilhaft auch weitere lipophile Komponenten - wie beispielsweise lipophile Wirkstoffe - enthalten.

Besonders vorteilhafte bei Raumtemperatur flüssige UV-Filtersubstanzen im Sinne der vorliegenden Erfindung sind Homomenthylsalicylat (INCl: Homosalate), 2-Ethylhexyl-2-cyano-3,3-diphenylacrylat (INCl: Octocrylene), 2-Ethylhexyl-2-hydroxybenzoat (2-Ethylhexylsalicylat, Octylsalicylat, INCl: Octyl Salicylate) und Ester der Zimtsäure, vorzugsweise 4-Methoxyzimtsäure(2-ethylhexyl)ester (2-Ethylhexyl-4-methoxycinnamat, INCl: Octyl Methoxycinnamate) und 4-Methoxyzimtsäureisopentylester (Isopentyl-4-methoxycinnamat, INCl: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans (TiO₂) Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂ | Octyltrimethylsilan | Degussa |
| Tioveil AQ 10PG | Alumina / Silica | Solaveil / Uniquema |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex^{®} 9020 verkauft wird.

Vorteilhafte weitere UV-Filtersubstanzen im Sinne der vorliegenden Erfindung sind sulfonierte, wasserlösliche UV-Filter, wie z. B.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCl-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCl Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCl-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCl: Methylene Bis-Benzotriazolmethylbutylphenol), welches unter der Handelsbezeichnung Tinosorb® S bei der CIBA-Chemikalien GmbH erhältlich ist;
- Dioctylbutylamidotriazon (INCl: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCl: Bisoctyltriazol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich sein. Vorteilhafte öllösliche Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

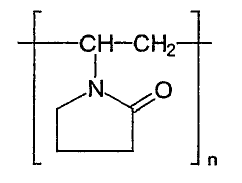

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### 1. O/W Sonnenschutz Emulsionen

| | **1** | **2** | **3** | **4** | **5** | **6** | **7** |
|---|---|---|---|---|---|---|---|
| Glycerinmonostearat SE | 0,50 | 1,00 | 3,00 | | | 1,50 | |
| Glyceryl Stearat Citrat | 2,00 | | | 1,00 | 2,00 | | 2,50 |
| Stearinsäure | | 3,00 | | 2,00 | | | |
| PEG-40 Stearat | 0,50 | | | | | 2,00 | |
| Cetyl Phosphat | | | | | 1,00 | | |
| Stearyl Alkohol | | | 3,00 | | | 2,00 | 0,50 |
| Cetyl Alkohol | 2,50 | 1,00 | | 1,50 | 0,50 | | 2,00 |
| Ethylhexyl Methoxycinnamat | | | | 5,00 | 6,00 | | 8,00 |
| Bis-Ethylhexyloxyphenol methoxyphenyl Triazin | | 1,50 | | 2,00 | 2,50 | | 2,50 |
| Butyl Methoxydibenzoylmethan | 1,00 | 3,00 | | 1,50 | 2,80 | 2,00 | 1.50 |
| Dinatrium Phenyl Dibenzimidazol Tetrasulfonat | 2,50 | 0.50 | | 2,00 | 1,00 | 1,70 | 0,30 |
| Ethylhexyl Triazon | 4,00 | | | 4,00 | 4,00 | 2,00 | |
| 4-Methylbenzyliden Campher | 4,00 | 4,00 | | | 2,00 | 4,00 | 2,00 |
| Octocrylen | | 4,00 | | | | | 2,50 |
| Diethylhexyl Butamido Triazon | 1,00 | | | 2,00 | 1,00 | | |
| Phenylbenzmidazol Sulfonsäure | 0,50 | | | 3,00 | | | |
| Methylen Bis-Benzotriazolyl Tetramethylbutylphenol | 2,00 | | | 1,50 | 2,50 | | |
| Benzophenon-3 | | | | 5,50 | | | |
| Isoamyl p-Methoxyzinnamat | | 1,50 | | | | | |
| Homosalat | | 2,00 | | | | | |
| Ethylhexylsalicylat | | | | | | | 5,00 |
| Drometrizol Trisiloxan | | | | | | 1,00 | |
| Terephthaliden Dicamphor Sulfonsäure | | 1,50 | | | 1,00 | 0,50 | |
| Diethylhexyl-2,6-naphthalat | 3,50 | 4,80 | 7,00 | 9,50 | 6,70 | 5,50 | 8,00 |
| Repellent 3535 | 5,0 | 9,5 | 25 | 15,5 | 10 | | 18 |
| Bayrepel KBR 3023 | | | | | | 12 | |
| Titandioxid MT-100Z | 1,00 | 1,50 | | 3,00 | 2,00 | 2,00 | |
| Zinkoxid HP1 | | | | 1,00 | | 2,00 | 3,00 |
| C12-15 Alkyl Benzoat | | 2,50 | | | 4,00 | 7,00 | 5,00 |
| Dicaprylyl Ether | | | 3,50 | | 2,00 | | |
| Butylenglycol Dicaprylat/Dicaprat | 5,00 | | | 6,00 | | | |
| Dicaprylyl Carbonat | | | 6,00 | | | 2,00 | 2,00 |
| Dimethicon | | 0,50 | 1,00 | | 2,00 | | |
| Cyclomethicon | 2,00 | | | 0,50 | | | 0,50 |
| Shea Butter | | 2,00 | | | | | 0,50 |
| PVP Hexadecen Copolymer | 0,50 | | | 0,50 | 1,00 | | 1,00 |
| Tricontanyl PVP | | 0,50 | 1,00 | | | | 1,00 |
| Glycerin | 3,00 | 7,50 | | 7,50 | 5,00 | | 2,50 |
| Xanthan Gummi | 0,15 | | 0,05 | | | | 0,30 |
| Sodium Carbomer | | 0,20 | 0,10 | 0,20 | | | |
| Vitamin E Acetat | 0,50 | | 0,25 | | 0,75 | | 1,00 |
| Polyurethan | | 0,50 | | 0,50 | | 1,00 | |
| Styrene/Acrylat Copolymer | 0.80 | | 3.00 | 1.50 | | | |
| DMDM Hydantoin | | 0,60 | 0,40 | 0,20 | | | |
| Konkaben LMB ® | | | | 0,18 | 0,20 | 0,10 | 0,15 |
| Methylparaben | 0,15 | | 0,25 | | 0,50 | | |
| Phenoxyethanol | 1,00 | 0,40 | | 0,40 | 0,50 | 0,40 | 0,60 |
| Ethanol | | 2,00 | 1,50 | | 3,00 | | 1,00 |
| Parfüm | 0,20 | 0,20 | 0,20 | 0,20 | 0,20 | 0,20 | 0,20 |
| Wasser | ad 100 | ad 100 | ad 100 | ad 100 | ad 100 | ad 100 | ad 100 |

### 2. Hydrodispersionen

| | **1** | **2** | **3** | 4 | 5 |
|---|---|---|---|---|---|
| Ceteareth-20 | 1,00 | | | 0,5 | |
| Cetyl Alkohol | | | 1,00 | | |
| Sodium Carbomer | | 0,20 | | 0,30 | |
| Acrylates/C10-30 Alkyl Acrylat Crosspolymer | 0,50 | | 0,40 | 0,10 | 0,10 |
| Xanthan Gummi | | 0,30 | 0,15 | | 0,50 |
| Ethylhexyl Methoxycinnamat | | | | | 8,00 |
| Bis-Ethylhexyloxyphenol methoxyphenyl Triazin | | 1,50 | | | 2,50 |
| Butyl Methoxydibenzoylmethan | 1,00 | 0.50 | 2,00 | | 2,50 |
| Dinatrium Phenyl Dibenzimidazol Tetrasulfonat | 0,50 | 1.80 | 1,50 | | 3.00 |
| Ethylhexyl Triazon | 4,00 | | 3,00 | | |
| 4-Methylbenzyliden Camphor | 4,00 | | | | 2,00 |
| Octocrylen | | 4,00 | 3,90 | | 2,50 |
| Diethyhexyl Butamido Triazon | 1,00 | | | | |
| Phenylbenzmidazol Sulfonsäure | 0,50 | | | | |
| Methylen Bis-Benzotriazolyl Tetramethylbuthylphenol | 2,50 | 0,50 | | | 0,80 |
| Drometrizol Trisiloxan | | | 1,00 | | 1,50 |
| Terephthaliden Dicamphor Sulfonsäure | | 0,50 | | | 1,00 |
| Diethylhexyl-2,6-naphthalat | 4,50 | 8,00 | 7,20 | 5,50 | 9,80 |
| Repellent 3535 | 5,0 | 12,0 | 5,0 | 22,5 | 18 |
| Bayrepel KBR 3023 | | | 15,5 | | |
| Titandioxid MT-100TV | 0,50 | | 2,00 | | 1,00 |
| Zinkoxid HP1 | | | 1,00 | | 3,00 |
| C12-15 Alkyl Benzoat | 2,00 | 2,50 | | | |
| Dicaprylyl Ether | | 4,00 | | | |
| Butylenglycol Dicaprylat/Dicaprat | 4,00 | | 2,00 | 6,00 | |
| Dicaprylyl Carbonat | | 2,00 | 6,00 | | |
| Dimethicon | | 0,50 | 1,00 | | |
| Phenyltrimethicon | 2,00 | | | 0,50 | 2,00 |
| Shea Butter | | 2,00 | | | |
| PVP Hexadecen Copolymer | 0,50 | | | 0,50 | 1,00 |
| Tricontanyl PVP | 0,50 | | 1,00 | | |
| Ethylhexylglycerin | | | 1,00 | | 0,50 |
| Glycerin | 3,00 | 7,50 | | 7,50 | 2,50 |
| Glycin Soja | | | 1,50 | | |
| Vitamin E Acetat | 0,50 | | 0,25 | | 1,00 |
| Polyurethan | | 0,60 | 1,50 | 1,00 | |
| Styrene/Acrylat Copolymer | | 2,50 | 0.50 | | 2.00 |
| DMDM Hydantoin | | 0,60 | 0,40 | 0,20 | |
| Konkaben LMB ® | 0,20 | | | | 0,15 |
| Methylparaben | 0,50 | | 0,25 | 0,15 | |
| Phenoxyethanol | 0,50 | 0,40 | | 1,00 | 0,60 |
| Ethanol | 3,00 | 2,00 | 1,50 | | 1,00 |
| Parfüm | 0,20 | 0,20 | 0,20 | 0,20 | 0,20 |
| Wasser | ad 100 | ad 100 | ad 100 | ad 100 | ad 100 |

### 3. W/O Sonnenschutz Emulsionen

| | **1** | **2** | **3** | 4 | **5** |
|---|---|---|---|---|---|
| Cetyldimethicon Copolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| Ethylhexyl Methoxycinnamat | | 8,00 | 3,0 | 5,00 | 4,00 |
| Bis-Ethylhexyloxyphenol methoxyphenyl Triazin | 2,00 | 2,50 | | 2,00 | 2,50 |
| Butyl Methoxydibenzoylmethan | 0.50 | 3,00 | | 1,00 | 0,70 |
| Dinatrium Phenyl Dibenzimidazol Tetrasulfonat | 0,50 | 1,00 | | 2,00 | 2.60 |
| Ethylhexyl Triazon | | | | 4,00 | |
| 4-Methylbenzyliden Camphor | | 2,00 | | 4,00 | 2,00 |
| Octocrylen | 0,90 | 2,50 | 3,90 | | 2,50 |
| Diethyhexyl Butamido Triazon | 1,00 | | | 2,00 | |
| Phenylbenzmidazol Sulfonsäure | 0,50 | | | 3,00 | 2,00 |
| Methylen Bis-Benzotriazolyl | | | | 0,50 | |
| Tetramethylbutylphenol | | | | | |
| Drometrizol Trisiloxan | | 1,00 | | | 1,50 |
| Terephthaliden Dicamphor Sulfonsäure | | | 1,00 | | 0,50 |
| Diethylhexyl-2,6-naphthalat | 7.50 | 5.50 | 3.50 | 8.80 | 9.70 |
| Repellent 3535 | 8,0 | 12,5 | | 20,5 | 25 |
| Bayrepel KBR 3023 | | | 6,0 | | |
| Titandioxid T805 | | 2,00 | 1,50 | | 3,00 |
| Zinkoxid NDM | 1,00 | | 3,00 | | 2,00 |
| Mineralöl | | | 10,0 | | 8,00 |
| C12-15 Alkyl Benzoat | | | | 9,00 | |
| Dicaprylyl Ether | 10,00 | | | | 7,00 |
| Butylenglycol Dicaprylat/Dicaprat | | | 2,00 | 8,00 | 4,00 |
| Dicaprylyl Carbonat | 5,00 | | 6,00 | | |
| Dimethicon | | 4,00 | 1,00 | 5,00 | |
| Cyclomethicon | 2,00 | 25,00 | | | 2,00 |
| Shea Butter | | | 3,00 | | |
| PVP Hexadecen Copolymer | 0,50 | | | 0,50 | 1,00 |
| Tricontanyl PVP | | | 0,50 | 1,00 | 0,50 |
| Ethylhexylglycerin | | 0,30 | 1,00 | | 0,50 |
| Glycerin | 3,00 | 7,50 | | 7,50 | 2,50 |
| Glycine Soja | | 1,00 | 1,50 | | |
| MgSO₄ | 1,00 | 0,50 | | 0,50 | |
| MgCl₂ | | | 1,00 | | 0,70 |
| Vitamin E Acetat | 0,50 | | 0,25 | | 1,00 |
| Styrene/Acrylat Copolymer | 0.50 | | | 2.50 | |
| Polyurethan | | 0,50 | | 1,50 | |
| DMDM Hydantoin | | 0,60 | 0,40 | 0,20 | |
| Methylparaben | 0,50 | | 0,25 | 0,15 | |
| Phenoxyethanol | 0,50 | 0,40 | | 1,00 | 0,60 |
| Ethanol | 3,00 | | 1,50 | | 1,00 |
| Parfüm | 0,20 | 0,20 | 0,20 | 0,20 | 0,20 |
| Wasser | ad 100 | ad 100 | ad 100 | ad 100 | ad 100 |

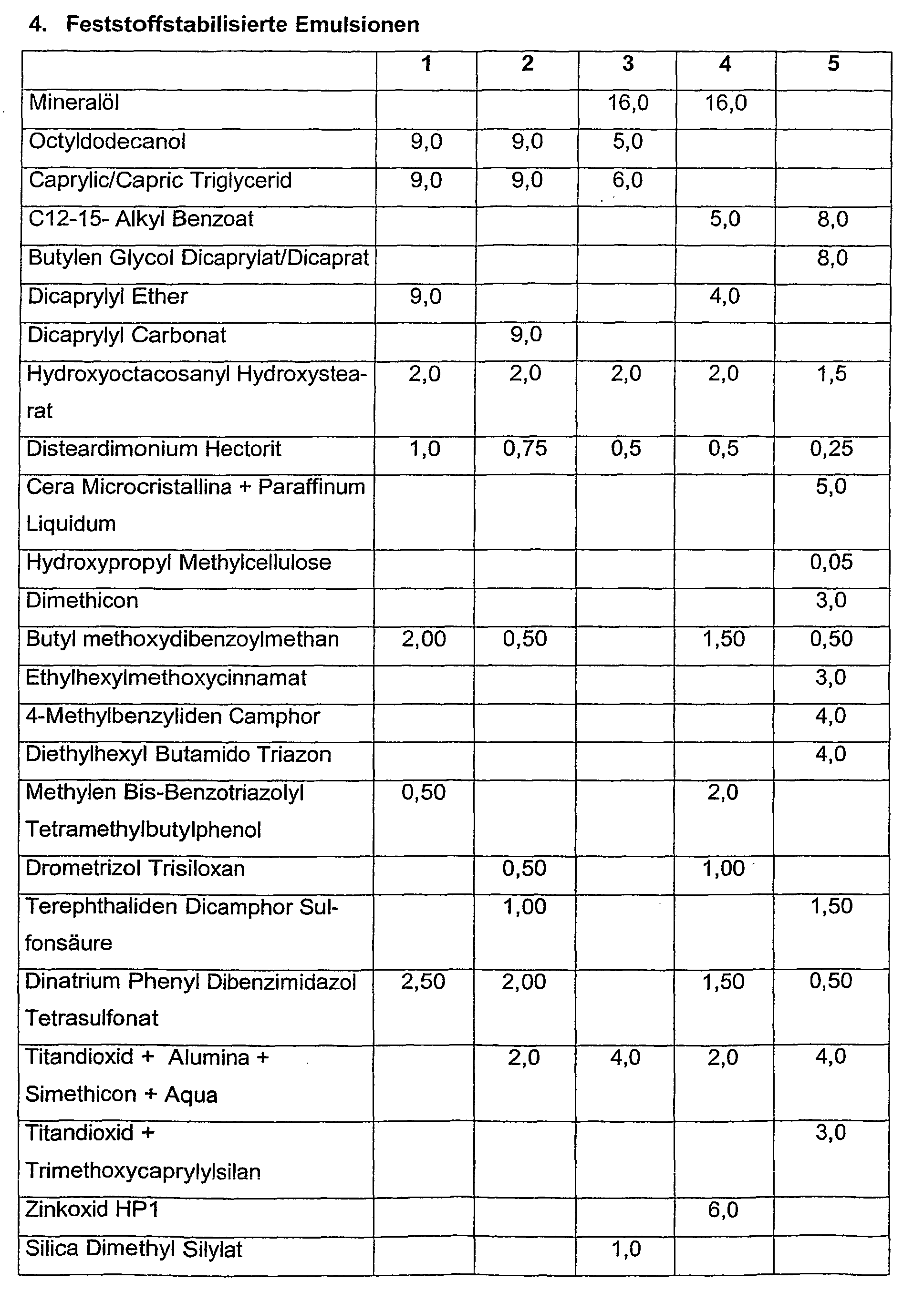

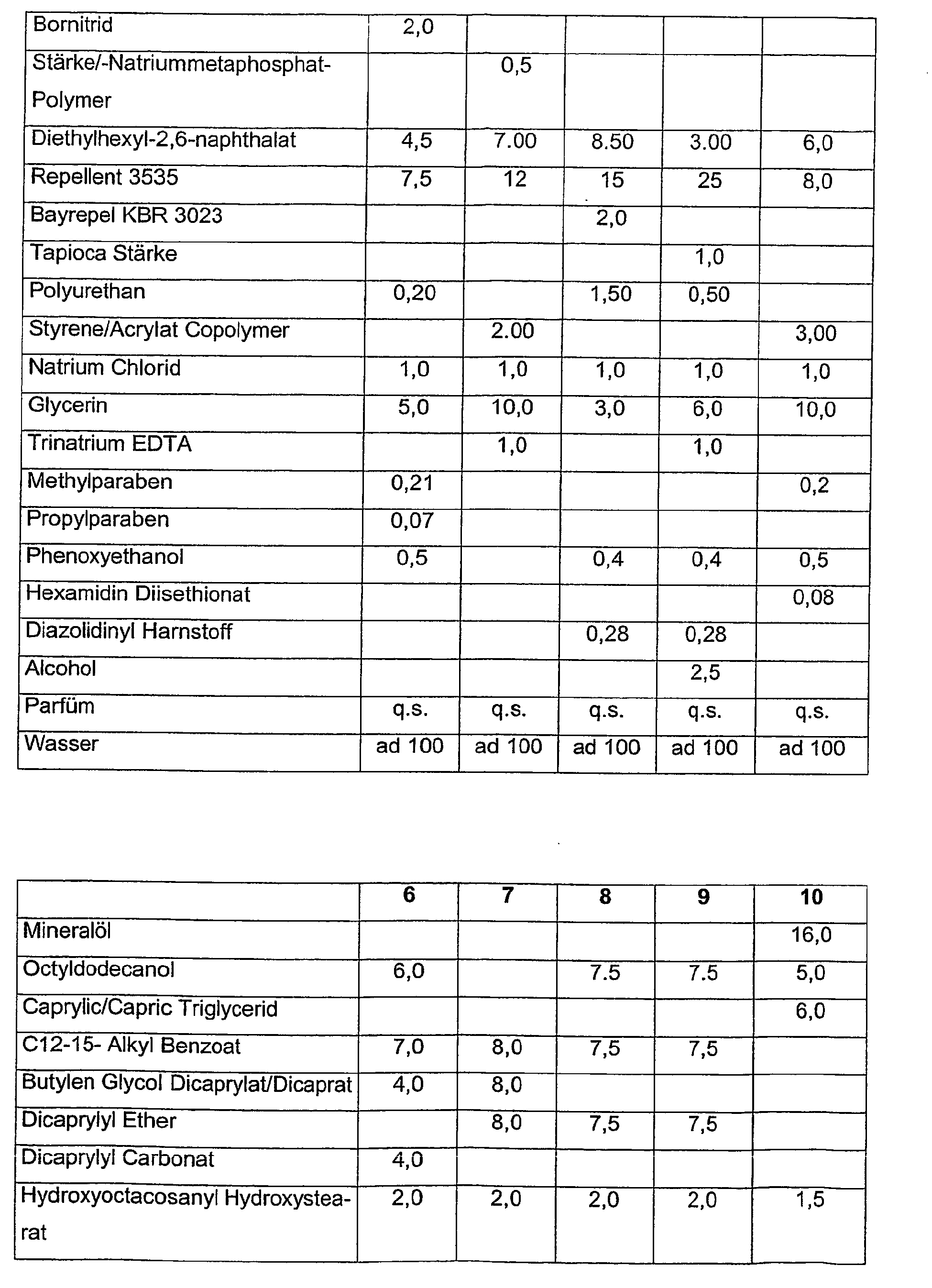

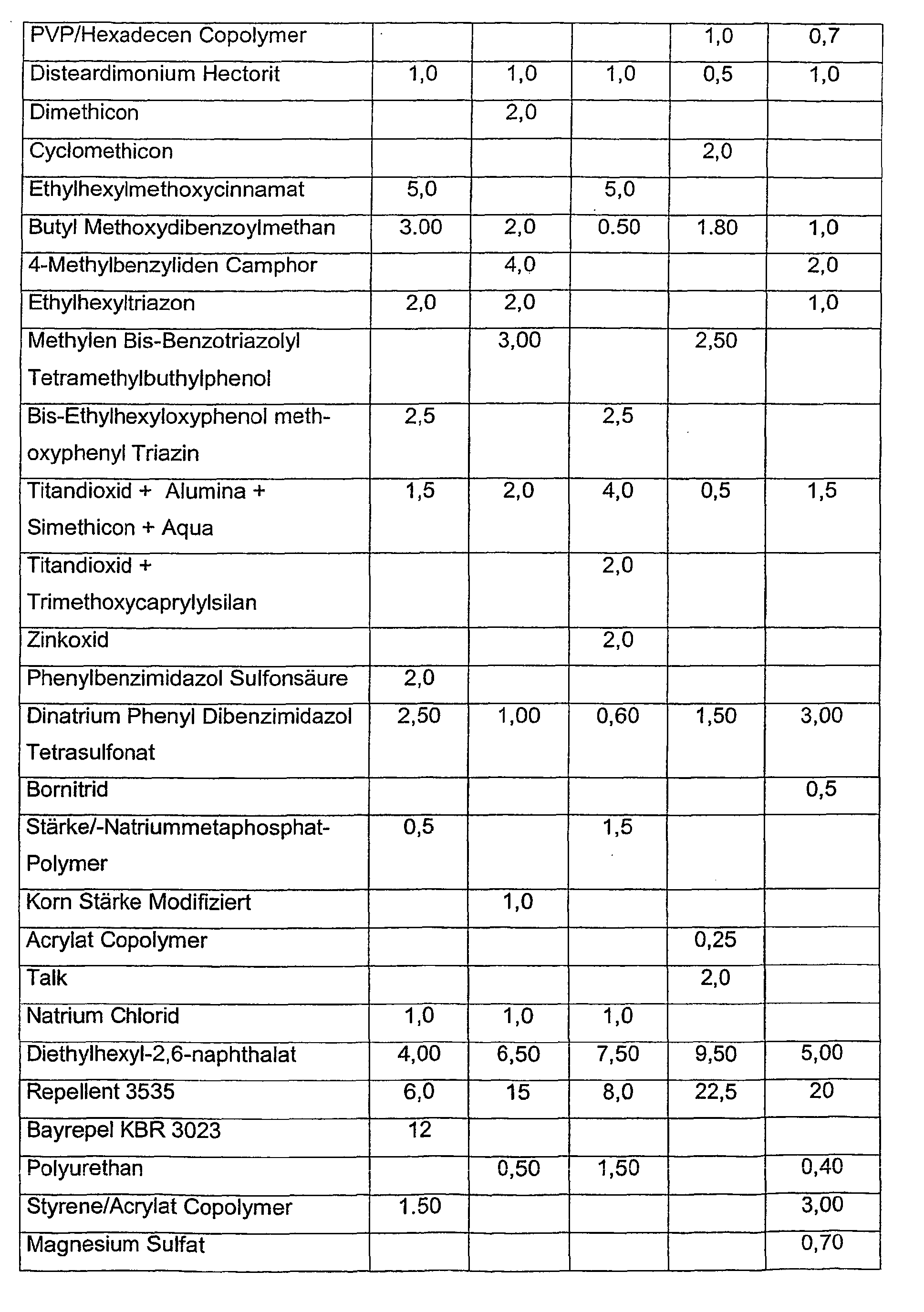

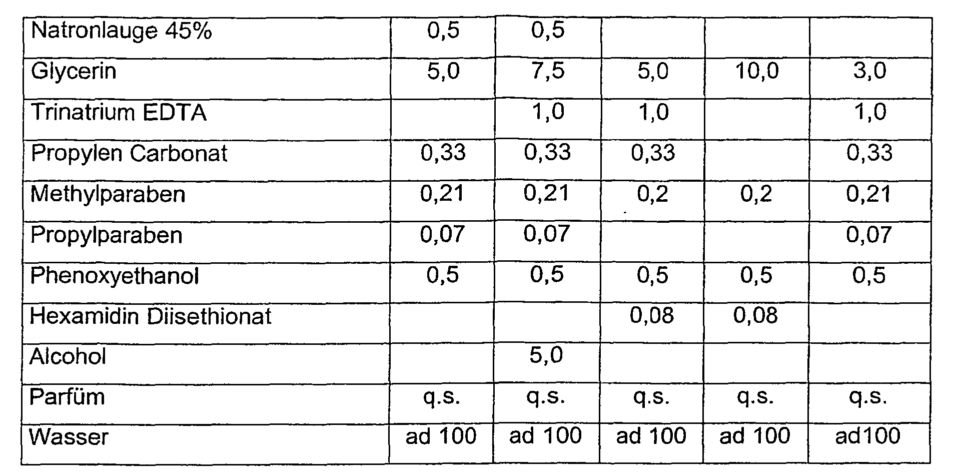

## Patentansprüche

1. Kosmetische und dermatologische Formulierungen mit mindestens einem Repellent-Wirkstoff, **dadurch gekennzeichnet, daß** sie mindestens ein Dialkylnaphthalat, welches sich durch die Strukturformel 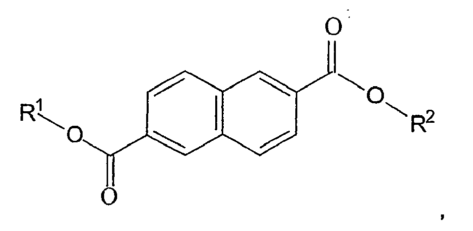 auszeichnet, worin R¹ und R² unabhängig voneinander gewählt werden aus der Gruppe der verzweigten und unverzweigten Alkylgruppen mit 6 bis 24 Kohlenstoffatomen, enthalten.

2. Zubereitung nach Anspruch 1, **dadurch gekennzeichnet, daß** der Gehalt an einem oder mehreren Dialkylnaphthalaten aus dem Bereich von 0,001 bis 20 Gew.-%, vorteilhaft 0,01 bis 15 Gew.-%, besonders bevorzugt 3 bis 10 Gew.-% gewählt wird, jeweils bezogen auf das Gesamtgewicht der Zubereitung.

3. Zubereitung nach einem der vorhergehenden Ansprüche, **dadurch gekennzeichnet, daß** der oder die Repellent-Wirkstoff(e) (eine oder mehrere Verbindungen) aus der Gruppe N,N-Diethyl-3-methylbenzamid, 3-(N-n-Butyl-N-acetyl-amino)propionsäureethylester und Dimethylphthalat gewählt wird/werden.

4. Zubereitung nach einem der vorhergehenden Ansprüche, **dadurch gekennzeichnet, daß** sie mindestens eine UV-Filtersubstanz, gewählt aus der Gruppe Triazine, Benzotriazole und organische und/oder anorganische Pigmente, enthält.

5. Zubereitung nach einem der vorhergehenden Ansprüche, **dadurch gekennzeichnet, daß** sie mindestens eine UV-A-Filtersubstanz und/oder einen Breitbandfilter, gewählt aus der Gruppe Dibenzoylmethanderivate [insbesondere das 4-(tert.-Butyl)-4'-methoxydibenzoylmethan] und 2,4-Bis-{[4-(2-Ethyl-hexyloxy)-2-hydroxy]-phenyl}-6-(4-methoxyphenyl)-1,3,5-triazin, enthält, wobei die weiteren Filtersubstanzen jeweils einzeln oder in beliebigen Kombinationen miteinander vorliegen können.

6. Verwendung von mindestens einem Dialkylnaphthalat, welches sich durch die Strukturformel 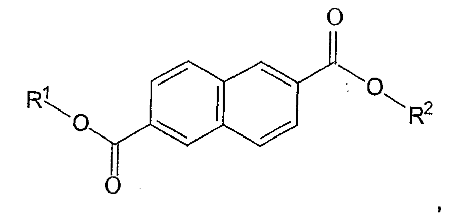 auszeichnet, worin R¹ und R² unabhängig voneinander gewählt werden aus der Gruppe der verzweigten und unverzweigten Alkylgruppen mit 6 bis 24 Kohlenstoffatomen, zur Erhöhung der Stabilität von kosmetischen Zubereitungen, welche mindestens einen Repellent-Wirkstoff enthalten.

7. Verwendung von mindestens einem Dialkylnaphthalat, welches sich durch die Strukturformel 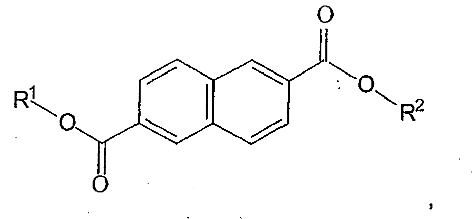 auszeichnet, worin R¹ und R² unabhängig voneinander gewählt werden aus der Gruppe der verzweigten und unverzweigten Alkylgruppen mit 6 bis 24 Kohlenstoffatomen, zur Herstellung von dermatologischen Zubereitungen, mit erhöhter Stabilität, welche mindestens einen Repellent-Wirkstoff enthalten.

## Claims

1. Cosmetic and dermatological formulation having at least one repellent active compound, **characterized in that** it comprises at least one dialkyl naphthalate which is distinguished by the structural formula 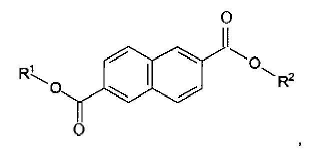 wherein R¹ and R² independently of one another are chosen from the group consisting of branched and unbranched alkyl groups having 6 to 24 carbon atoms.

2. Formulation according to Claim 1, **characterized in that** the content of one or more dialkyl naphthalates is chosen from the range of 0.001 to 20 wt.%, advantageously 0.01 to 15 wt.%, particularly preferably 3 to 10 wt.%, in each case based on the total weight of the formulation.

3. Formulation according to one of the preceding claims, **characterized in that** the repellent active compound(s) (one or more compounds) is/are chosen from the group consisting of N,N-diethyl-3-methylbenzamide, 3-(N-n-butyl-N-acetyl-amino)proplonic acid ethyl ester and dimethyl phthalate.

4. Formulation according to one of the preceding claims, **characterized in that** it comprises at least one UV filter substance chosen from the group consisting of triazines, benzotriazoles and organic and/or inorganic pigments.

5. Formulation according to one of the preceding claims, **characterized in that** it comprises at least one UV-A filter substance and/or a broad-band filter, chosen from the group consisting of dibenzoylmethane derivatives [in particular 4-(tert-butyl)-4'-methoxydibenzoylmethane] and 2,4-bis-{[4-(2-ethyl-hexylnxy)-2-hydroxy]-phenyl}-6-(4-methoxyphenyl)-1,3,5-triazine, it being possible for the further filter substances to be present in each case individually or in any desired combinations with one another.

6. Use of at least one dialkyl naphthalate which is distinguished by the structural formula 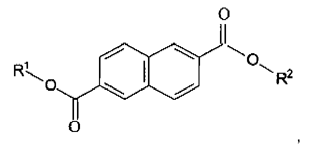 wherein R¹ and R² independently of one another are chosen from the group consisting of branched and unbranched alkyl groups having 6 to 24 carbon atoms, for increasing the stability of cosmetic formulations comprising at least one repellent active compound.

7. Use of at least one dialkyl naphthalate which is distinguished by the structural formula 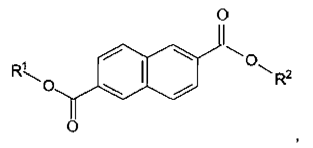 wherein R¹ and R² independently of one another are chosen from the group consisting of branched and unbranched alkyl groups having 6 to 24 carbon atoms, for in the manufacture of dermatological formulations having increased stability and comprising at least one repellent active compound.

## Revendications

1. Compositions cosmétiques et dermatologiques comportant au moins un actif insectifuge, **caractérisées en ce qu'**elles contiennent au moins un naphtalate de dialkyle qui se distingue par la formule développée 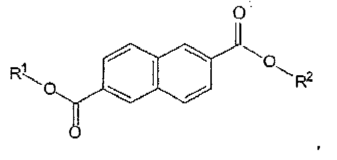 dans laquelle R¹ et R² sont choisis, indépendamment l'un de l'autre, dans l'ensemble des groupes alkyle ramifiés et non ramifiés ayant de 6 à 24 atomes de carbone.

2. Préparation selon la revendication 1, **caractérisée en ce que** la teneur en un ou plusieurs naphtalates de dialkyle est choisie dans la plage allant de 0,001 à 20 % en poids, avantageusement de 0,01 à 15 % en poids, de façon particulièrement préférée de 3 à 10 % en poids, chaque fois par rapport au poids total de la préparation.

3. Préparation selon l'une quelconque des revendications précédentes, **caractérisée en ce que** l'actif insectifuge ou les actifs insectifuges (un ou plusieurs composés) est(sont) choisi(s) dans l'ensemble constitué par le N,N-diéthyl-3-méthylbenzamide, le 3-(N-n-butyl-N-acétyl-amino)-propionate d'éthyle et le phtalate de diméthyle.

4. Préparation selon l'une quelconque des revendications précédentes, **caractérisée en ce qu'**elle contient au moins un filtre UV, choisi dans l'ensemble constitué par les triazines, les benzotriazoles et les pigments organiques et/ou minéraux.

5. Préparation selon l'une quelconque des revendications précédentes, **caractérisée en ce qu'**elle contient au moins un filtre UV-A et/ou un filtre à large bande, choisi(s) dans l'ensemble constitué par des dérivés de dibenzoylméthane [en particulier le 4-(tert-butyl)-4'-méthoxydibenzoylméthane] et la 2,4-bis-{[4-(2-éthyl-hexyloxy)-2-hydroxy]-phényl}-6-(4-méthoxyphényl)-1,3,5-triazine, les autres filtres pouvant dans chaque cas être présents individuellement ou en associations quelconques les uns avec les autres.

6. Utilisation d'au moins un naphtalate de dialkyle qui se distingue par la formule développée 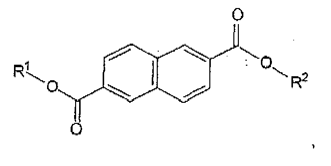 dans laquelle R¹ et R² sont choisis, indépendamment l'un de l'autre, dans l'ensemble des groupes alkyle ramifiés et non ramifiés ayant de 6 à 24 atomes de carbone, pour augmenter la stabilité de préparations cosmétiques qui contiennent au moins un actif insectifuge.

7. Utilisation d'au moins un naphtalate de dialkyle qui se distingue par la formule développée 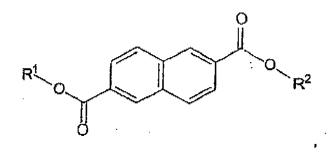 dans laquelle R¹ et R² sont choisis, indépendamment l'un de l'autre, dans l'ensemble des groupes alkyle ramifiés et non ramifiés ayant de 6 à 24 atomes de carbone, pour la fabrication de préparations dermatologiques à stabilité accrue, qui contiennent au moins un actif insectifuge.
